(19) Europäisches Patentamt
European Patent Office
Office européen des brevets

(11) Numéro de publication: **0 119 896**
**B1**

(12) # FASCICULE DE BREVET EUROPEEN

(45) Date de publication du fascicule du brevet: **08.10.86**

(51) Int. Cl.⁴: **C 07 D 339/04**, C 07 D 409/04, A 61 K 31/385

(21) Numéro de dépôt: **84400368.1**

(22) Date de dépôt: **23.02.84**

(54) **Nouveaux dérivés de l'amino-5 dithiole-1,2 one-3, leur préparation et les compositions médicinales qui les contiennent.**

(30) Priorité: **24.02.83 FR 8303005**

(43) Date de publication de la demande: **26.09.84 Bulletin 84/39**

(45) Mention de la délivrance du brevet: **08.10.86 Bulletin 86/41**

(84) Etats contractants désignés: **AT BE CH DE FR GB IT LI LU NL SE**

(56) Documents cités:
**FR - A - 1 498 374**
**FR - A - 2 347 928**

(73) Titulaire: **RHONE-POULENC SANTE, Les Miroirs 18 Avenue d'Alsace, F-92400 Courbevoie Cedex (FR)**

(72) Inventeur: **Bourzat, Jean-Dominique, 35, rue Santos-Dumont, F-75015 Paris (FR)**
Inventeur: **Cotrel, Claude, 17 A, avenue du Dr. Arnold Netter, F-75012 Paris (FR)**
Inventeur: **Farge, Daniel, 30, rue des Pins Sylvestres, F-94320 Thiais (FR)**
Inventeur: **Paris, Jean-Marc, 8, rue des Acacias, F-77360 Vaires S/Marne (FR)**
Inventeur: **Taurand, Gérard, 13, Passage Saillenfait, F-94000 Creteil (FR)**

(74) Mandataire: **Le Goff, Yves et al, RHONE-POULENC RECHERCHES Brevets Pharma 25, Quai Paul Doumer, F-92408 Courbevoie (FR)**

EP 0 119 896 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouveaux dérivés de l'amino-5 dithiole-1,2 one-3 de formule générale:

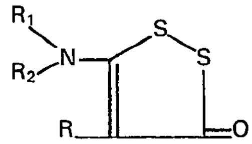

(I)

dans laquelle R représente un atome d'hydrogène ou de chlore et

— soit $R_1$ et $R_2$ forment ensemble avec l'atome d'azote auquel ils sont liés un cycle tétrahydro-1,2,3,4 quinoxaline, tétrahydro-1,2,3,4 quinoléine, tétrahydro-1,2,3,4 isoquinoléine, dihydro-3,4 2H-benzoxazine-1,4, tétrahydro-2,3,4,5 1H-benzazépine ou isoindoline, tous ces cycles pouvant éventuellement porter un ou plusieurs substituants en position quelconque choisis parmi les atomes d'halogène et les radicaux carboxy, alcoyloxycarbonyle, carbamoyle, alcoylcarbamoyle, dialcoylcarbamoyle, trifluorométhyle, cyano, nitro, alcoyloxy, alcoylthio ou alcoyle éventuellement substitué par un radical carboxy, alcoyloxycarbonyle, carbamoyle, alcoylcarbamoyle, dialcoylcarbamoyle, hydroxy, amino, alcoylamino ou dialcoylamino,

— soit $R_1$ représente un radical alcoyle et $R_2$ représente un radical alcoyle substitué par un radical phényle lui-même non substitué ou substitué par un ou plusieurs atomes ou radicaux choisis parmi les atomes d'halogène et les radicaux alcoyle, alcoyloxy, alcoylthio, hydroxy, amino, alcoylamino, dialcoylamino, carboxy, alcoyloxycarbonyle, carbamoyle, alcoylcarbamoyle, dialcoylcarbamoyle, trifluorométhyle, cyano ou nitro,

étant entendu que tous les radicaux alcoyle et portions alcoyle cités ci-dessus ou qui seront cités dans la suite, contiennent 1 à 4 atomes de carbone en chaîne droite ou ramifiée,

ainsi que, lorsqu'ils existent, leurs sels d'addition avec les acides, leurs sels métalliques et leurs sels d'addition avec les bases azotées.

Selon l'invention, les produits de formule générale (I) dans laquelle R représente un atome de chlore et les autres symboles sont définis comme précédemment peuvent être préparés par action d'une amine de formule générale:

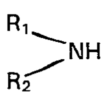

(II)

dans laquelle $R_1$ et $R_2$ sont définis comme précédemment, sur la dichloro-4,5 dithiole-1,2 one-3 de formule:

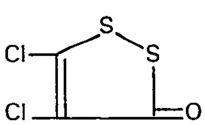

(III)

La réaction s'effectue généralement dans un solvant organique, tel qu'un alcool comme le méthanol, ou le diméthylformamide en présence d'un léger excès par rapport à la quantité théorique d'un accepteur d'acide tel qu'un carbonate ou bicarbonate alcalin comme le carbonate ou le bicarbonate de potassium, ou une base organique telle que le nitrilo-tris-(propanol-2) à une température comprise entre 0 et 80°C, de préférence voisine de 20°C.

La dichloro-4,5 dithiole-1,2 one-3 de formule (III) peut être préparée selon la méthode de F. BOBERG, Ann. Chem. 681, 169 (1965).

Les amines de formule générale (II) peuvent être préparées par utilisation ou adaptation des méthodes connues dans l'art antérieur à la portée de l'homme du métier.

Selon l'invention, les produits de formule générale (I) dans laquelle R représente un atome d'hydrogène et les autres symboles sont définis comme précédemment peuvent être préparés par déchloration d'un produit de formule générale (I) dans laquelle R représente un atome de chlore et les autres symboles sont définis comme précédemment, c'est-à-dire un produit de formule générale:

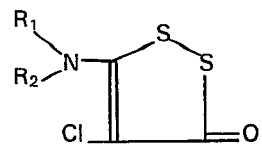

(IV)

La déchloration peut être effectuée par toute méthode connue de l'homme du métier pour effectuer cette réaction sans toucher au reste de la molécule. Il est particulièrement avantageux d'effectuer la déchloration au moyen d'un hydrure de trialcoylétain tel que l'hydrure de tributylétain à une température comprise entre 0 et 40°C.

Les nouveaux produits de formule générale (I) peuvent être purifiés par les méthodes connues habituelles par exemple par cristallisation, chromatographie ou, le cas échéant, extractions successives en milieu acide et basique.

Les nouveaux produits de formule générale (I) dans laquelle les substituants $R_1$, $R_2$ sont porteurs d'une fonction aminée tel qu'il est défini précédemment peuvent être éventuellement transformés en sels d'addition avec les acides par action d'un acide dans un solvant organique tel qu'un alcool, une cétone, un ester ou un solvant chloré. Le sel précipite, éventuellement après concentration de sa solution; il est séparé par filtration ou décantation.

De manière analogue, les produits de formule générale (I) dans laquelle les substituants $R_1$, $R_2$ sont porteurs d'une fonction acide tel qu'il est défini précédemment peuvent être éventuellement transformés en sels métalliques ou en sels d'addition avec les bases azotées.

Il est entendu pour l'homme du métier que, pour la mise en oeuvre des procédés selon l'invention décrits précédemment, il peut être nécessaire d'introduire des groupements protecteurs des fonctions présentes dans la molécule des produits réagissant, afin d'éviter les réactions secondaires avant de mettre en oeuvre l'un ou l'autre des procédés selon l'invention. Le groupement protecteur pourra ensuite être conservé ou éliminé selon que le produit obtenu est ou non un produit selon l'invention. Ainsi, lorsqu'est présente dans la molécule une fonction

amine, il est nécessaire de protéger cette fonction, par exemple par un radical tert-butoxycarbonyle puis de libérer la fonction amine en milieu acide (par exemple au moyen d'une solution aqueuse d'acide chlorhydrique ou mieux d'une solution acétique de gaz chlorhydrique). De même, lorsqu'est présente dans la molécule une fonction hydroxylée (alcool ou acide carboxylique), cette fonction est avantageusement protégée sous forme d'éther ou d'ester; la fonction initiale peut être régénérée selon les techniques habituelles connues de l'homme du métier.

On connaît déjà par les brevets allemands 1.242.324 et 1.278.701 et par le certificat d'addition n° 94485 au brevet français 1.498.374 des amino-5 chloro-4 dithiole-1,2 one-3; aucun de ces documents ne divulgue les produits selon l'invention ni ne suggère une application thérapeutique pour ce type de produits.

Les nouveaux produits selon l'invention et leurs sels, lorsqu'ils existent, présentent d'intéressantes propriétés pharmacologiques les rendant susceptibles d'application dans le traitement de fond de la maladie rhumatismale. Ils se sont montrés actifs à des concentrations voisines de $5 \times 10^{-6}$ M dans le test de la mesure de l'activation des macrophages in vitro, selon J. SCHNYDER et M. BAGGIOLINI (J. Exp. Med. 148, 1449, 1978).

En outre, les produits de formule générale (I) présentent une faible toxicité. Leur $DL_{50}$ est généralement supérieure à 900 mg/kg par voie orale chez la souris en administration unique.

Sont particulièrement intéressants les produits de formule générale (I) dans laquelle R représente un atome de chlore et

— soit $R_1$ et $R_2$ forment ensemble avec l'atome d'azote auquel ils sont liés un cycle tétrahydro-1,2,3,4 quinoxaline, tétrahydro-1,2,3,4 quinoléine, tétrahydro-1,2,3,4 isoquinoléine, dihydro-3,4 2H-benzoxazine-1,4, tétrahydro-2,3,4,5 1H-benzazépine ou isoindoline, tous ces cycles pouvant éventuellement porter un substituant alcoyle,

— soit $R_1$ représente un radical alcoyle et $R_2$ représente un radical alcoyle substitué par un radical phényle.

Pour l'emploi médicinal, il peut être fait usage des produits selon l'invention tels quels ou, lorsqu'ils existent, à l'état de sels pharmaceutiquement acceptables, c'est-à-dire non toxiques aux doses d'utilisation.

Comme sels pharmaceutiquement acceptables, on peut citer les sels d'addition avec les acides minéraux tels que chlorhydrates, sulfates, nitrates, phosphates ou organiques tels que acétates, propionates, succinates, benzoates, fumarates, maléates, méthanesulfonates, iséthionates, théophilline-acétates, salicylates, phénolphtalinates, méthylène-bis-β-oxynaphtoates ou des dérivés de substitution de ces composés. Comme sels pharmaceutiquement acceptables, on peut encore citer les sels avec les métaux alcalins tels que le sodium, le potassium ou le lithium, avec les métaux alcalinoterreux tels que les sels de calcium et de magnésium, et les sels d'addition avec les bases organiques tels que les sels d'éthanolamine et de lysine.

Les exemples suivants, donnés à titre non limitatif, montrent comment l'invention peut être mise en pratique.

*Exemple 1*

Une suspension de 5,5 g de bicarbonate de potassium dans une solution de 9,35 g de dichloro-4,5 dithiole-1,2 one-3 et de 8,14 g de méthyl-1 tétrahydro-1,2,3,4 quinoxaline dans 75 $cm^3$ de méthanol est agitée pendant 20 heures à une température voisine de 20°C. Le produit insoluble est séparé par filtration, lavé 3 fois par 30 $cm^3$ au total de méthanol puis 5 fois par 100 $cm^3$ au total d'eau distillée et séché à l'air à une température voisine de 20°C. Après recristallisation dans l'acétonitrile, on obtient 10,3 g de chloro-4 (méthyl-4 tétrahydro-1,2,3,4 quinoxalinyl-1)-5 dithiole-1,2 one-3 fondant à 132°C.

La méthyl-1 tétrahydro-1,2,3,4 quinoxaline peut être préparée selon la méthode décrite par R.F. SMITH, W.J. REBEL et T.H. BEACH, J. Org. Chem., 24, 205, (1959).

*Exemple 2*

En opérant d'une manière analogue à celle décrite à l'exemple 1, mais à partir de 9,35 g de dichloro-4,5 dithiole-1,2 one-3 et de 7,3 g de tétrahydro-1,2,3,4 quinoléine, on obtient, après recristallisation dans l'acétonitrile, 8,9 g de chloro-4 (tétrahydro-1,2,3,4 quinolyl)-5 dithiole-1,2 one-3 fondant à 120°C.

*Exemple 3*

En opérant d'une manière analogue à celle décrite à l'exemple 1, mais à partir de 11,2 g de dichloro-4,5 dithiole-1,2 one-3 et de 8,9 g de dihydro-3,4 2H-benzoxazine-1,4, on obtient, après recristallisation dans l'acétonitrile, 10,2 g de chloro-4 (dihydro-3,4 2H-benzoxazine-1,4 yl-4)-5 dithiole-1,2 one-3 fondant à 143°C.

La dihydro-3,4 2H-benzoxazine-1,4 peut être préparée par la méthode décrite par H. SHIRAI, T. HAYAZAKI et H. YASUO, Chem. Abstr. 73 25378 g.

*Exemple 4*

En opérant d'une manière analogue à celle décrite à l'exemple 1, mais à partir de 9,3 g de dichloro-4,5 dithiole-1,2 one-3 et 7,3 g de tétrahydro-1,2,3,4 isoquinoléine, on obtient, après recristallisation dans l'acétonitrile, 10,8 g de chloro-4 (tétrahydro-1,2,3,4 isoquinolyl-2)-5 dithiole-1,2 one-3 fondant à 114°C.

*Exemple 5*

En opérant d'une manière analogue à celle décrite à l'exemple 1, mais à partir de 7,8 g de dichloro-4,5 dithiole-1,2 one-3 et de 5 g d'isoindoline, on obtient, après recristallisation dans le dioxanne, 6,9 g de chloro-4 (isoindolinyl-2)-5 dithiole-1,2 one-3 fondant à 194°C.

L'isoindoline peut être préparée selon la méthode décrite par J. BORNSTEIN, S.C. LASHUA et A.P. BOISELLE, J. Org. Chem. 22, 1255 (1957).

*Exemple 6*

En opérant d'une manière analogue à celle décrite à l'exemple 1, mais à partir de 3,16 g de dichloro-4,5 dithiole-1,2 one-3 et de 2,5 g de tétrahydro-2,3,4,5 1H-benz[d]azépine, on obtient après recristallisation

dans l'acétonitrile, 2,3 g de chloro-4 (tétrahydro-2,3,4,5 1H-benz[d]azépinyl-3)-5 dithiole-1,2 one-3 sous forme d'une poudre ocre fondant à 141 °C.

La tétrahydro-2,3,4,5 1H-benz[d]azépine peut être préparée selon la méthode décrite par P. RUGGLI, B.B. BUSSEMAKER, W. MÜLLER et A. STAUB, Helv. Chim. Acta, 18, 1388 (1935).

*Exemple 7*

A une solution de 11,2 g de dichloro-4,5 dithiole-1,2 one-3 dans 350 $cm^3$ de méthanol, on ajoute 6 g de bicarbonate de potassium puis une solution de 8 g de N-méthyl phénéthylamine dans 150 $cm^3$ de méthanol. Le mélange réactionnel est agité pendant 20 heures à une température voisine de 20°C, puis filtré et concentré à sec sous pression réduite (2,7 kPa) à 40°C. Le résidu est dissous dans 210 $cm^3$ de chlorure de méthylène; la solution obtenue est lavée 3 fois par 60 $cm^3$ au total d'eau distillée, séchée sur du sulfate de magnésium, filtrée et concentrée à sec sous pression réduite (2,7 kPa) à 40°C. Les cristaux obtenus sont recristallisés dans un mélange d'éther isopropylique et d'acétate d'éthyle (75-25 en volumes); on obtient ainsi 5,3 g de chloro-4 [N-méthyl N-(phényl-2 éthyl) amino]-5 dithiole-1,2 one-3 sous forme de cristaux jaunes fondant à 75°C.

*Exemple 8*

En opérant d'une manière analogue à celle décrite à l'exemple 1, mais à partir de 11,7 g de dichloro-4,5 dithiole-1,2 one-3 et de 7,6 g de N-méthyl benzylamine, on obtient 15,6 g de produit brut huileux. Ce produit est dissous dans 50 $cm^3$ de chlorure de méthylène et la solution obtenue est versée sur 450 g de gel de silice contenus dans une colonne de 6 cm de diamètre. On élue d'abord avec 2,7 litres d'un mélange de cyclohexane et d'acétate d'éthyle (70-30 en volumes); l'éluat correspondant est éliminé. On élue ensuite avec 3,5 litres du mélange utilisé précédemment; l'éluat correspondant est concentré à sec sous pression réduite (0,13 kPa) à 20°C. On obtient ainsi 12,3 g de chloro-4 (N-benzyl N-méthylamino)-5 dithiole-1,2 one-3 sous forme d'une huile jaune [Rf = 0,36; chromatographie sur couche mince de gel de silice; éluant cyclohexane-acétate d'éthyle (70-30 en volumes)].

La présente invention concerne également les médicaments constitués par un produit de formule générale (I), sous forme libre ou sous forme de sels d'addition avec un acide, de sels métalliques ou de sels d'addition avec une base pharmaceutiquement acceptables, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale, rectale ou topique.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes tels que amidon, cellulose, saccharose, lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les produits selon l'invention sont particulièrement utiles dans le traitement de fond de la maladie rhumatismale.

Les doses dépendent de l'effet recherché et de la durée du traitement; elles sont généralement comprises entre 50 et 1000 mg par jour par voie orale pour un adulte en une ou plusieurs prises.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre des compositions selon l'invention.

*Exemple*

On prépare, selon la technique habituelle, des comprimés dosés à 50 mg de produit actif ayant la composition suivante:

| | |
|---|---|
| — chloro-4 (tétrahydro-1,2,3,4 isoquinolyl-2)-5 dithiole-1,2 one-3 | 50 mg |
| — amidon | 60 mg |
| — lactose | 50 mg |
| — stéarate de magnésium | 2 mg |

**Revendications pour les Etats contractants:**
BE, CH, DE, FR, GB, IT, LI, LU, NL, SE

1. Dérivé de l'amino-5 dithiole-1,2 one-3, caractérisé en ce qu'il répond à la formule générale:

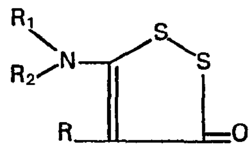

dans laquelle R représente un atome d'hydrogène ou de chlore et

— soit $R_1$ et $R_2$ forment ensemble avec l'atome d'azote auquel ils sont liés un cycle tétrahydro-1,2,3,4 quinoxaline, tétrahydro-1,2,3,4 quinoléine, tétrahydro-1,2,3,4 isoquinoléine, dihydro-3,4 2H-benzoxazine-1,4, tétrahydro-2,3,4,5 1H-benzazépine ou isoindoline, tous ces cycles pouvant éventuellement porter un ou plusieurs substituants en position quelconque choisis parmi les atomes d'halogène et les radicaux carboxy, alcoyloxycarbonyle, carbamoyle, alcoylcarbamoyle, dialcoylcarbamoyle, trifluorométhyle, cyano, nitro, alcoyloxy, alcoylthio ou alcoyle éventuellement substitué par un radical carboxy, alcoyloxycarbonyle, carbamoyle, alcoylcarbamoyle, dialcoylcarbamoyle, hydroxy, amino, alcoylamino ou dialcoylamino,

— soit $R_1$ représente un radical alcoyle et $R_2$ représente un radical alcoyle substitué par un radical phényle lui-même non substitué ou substitué par un ou plusieurs atomes ou radicaux choisis parmi les atomes d'halogène et les radicaux alcoyle, alcoyloxy, alcoylthio, hydroxy, amino, alcoylamino, dialcoylamino, carboxy, alcoyloxycarbonyle, carbamoyle, alcoylcarbamoyle, dialcoylcarbamoyle, trifluorométhyle, cyano, nitro,

étant entendu que tous les radicaux alcoyle et portions alcoyle cités ci-dessus contiennent 1 à 4 atomes de carbone en chaîne droite ou ramifiée,

ainsi que, lorsqu'ils existent, ses sels d'addition avec les acides, ses sels métalliques et ses sels d'addition avec les bases azotées.

2. Procédé de préparation d'un produit selon la revendication 1 dans la formule duquel R représente un atome de chlore et les autres symboles sont définis comme à la revendication 1, caractérisé en ce que l'on fait réagir une amine de formule générale:

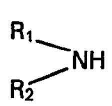

dans laquelle $R_1$ et $R_2$ sont définis comme à la revendication 1, sur la dichloro-4,5 dithiole-1,2 one-3 de formule:

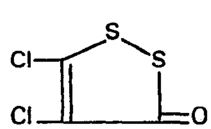

puis isole le produit obtenu et le transforme, le cas échéant, en un sel d'addition avec un acide, un sel métallique ou un sel d'addition avec une base azotée.

3. Procédé de préparation d'un produit selon la revendication 1 dans la formule duquel R représente un atome d'hydrogène et les autres symboles sont définis comme à la revendication 1, caractérisé en ce que l'on déchlore un produit de formule générale (I) dans la formule duquel R représente un atome de chlore et les autres symboles sont définis comme à la revendication 1, c'est-à-dire un produit de formule générale:

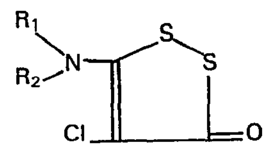

puis isole le produit obtenu et le transforme, le cas échéant, en un sel d'addition avec un acide, un sel métallique ou un sel d'addition avec une base azotée.

4. Procédé selon la revendication 3, caractérisé en ce que la déchloration est effectuée au moyen d'un hydrure de trialcoylétain.

5. Procédé selon l'une des revendications 3 ou 4, caractérisé en ce que la déchloration est effectuée au moyen de l'hydrure de tributylétain.

6. Composition pharmaceutique, caractérisée en ce qu'elle contient au moins un produit selon la revendication 1 associé avec un ou plusieurs diluants ou adjuvants compatibles et pharmaceutiquement acceptables.

**Revendications pour l'Etat contractant:** AT

1. Procédé de préparation d'un dérivé de l'amino-5 dithiole-1,2 one-3, de formule générale:

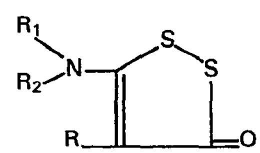

(I)

dans laquelle R représente un atome d'hydrogène ou de chlore et

— soit $R_1$ et $R_2$ forment ensemble avec l'atome d'azote auquel ils sont liés un cycle tétrahydro-1,2,3,4 quinoxaline, tétrahydro-1,2,3,4 quinoléine, tétrahydro-1,2,3,4 isoquinoléine, dihydro-3,4 2H-benzoxazine-1,4, tétrahydro-2,3,4,5 1H-benzazépine ou isoindoline, tous ces cycles pouvant éventuellement porter un ou plusieurs substituants en position quelconque choisis parmi les atomes d'halogène et les radicaux carboxy, alcoyloxycarbonyle, carbamoyle, alcoylcarbamoyle, dialcoylcarbamoyle, trifluorométhyle, cyano, nitro, alcoyloxy, alcoylthio ou alcoyle éventuellement substitué par un radical carboxy, alcoyloxycarbonyle, carbamoyle, alcoylcarbamoyle, dialcoylcarbamoyle, hydroxy, amino, alcoylamino ou dialcoylamino,

— soit $R_1$ représente un radical alcoyle et $R_2$ représente un radical alcoyle substitué par un radical phényle lui-même non substitué ou substitué par un ou plusieurs atomes ou radicaux choisis parmi les atomes d'halogène et les radicaux alcoyle, alcoyloxy, alcoylthio, hydroxy, amino, alcoylamino, dialcoylamino, carboxy, alcoyloxycarbonyle, carbamoyle, alcoylcarbamoyle, dialcoylcarbamoyle, trifluorométhyle, cyano, nitro,
étant entendu que tous les radicaux alcoyle et portions alcoyle cités ci-dessus contiennent 1 à 4 atomes de carbone en chaîne droite ou ramifiée,
ainsi que, lorsqu'ils existent, ses sels d'addition avec les acides, ses sels métalliques et ses sels d'addition avec les bases azotées, caractérisé en ce que
pour la préparation d'un produit de formule générale (I) dans laquelle R représente un atome de chlore et les autres symboles sont définis comme précédemment, on fait réagir une amine de formule générale:

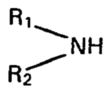

dans laquelle $R_1$ et $R_2$ sont définis comme précédemment sur la dichloro-4,5 dithiole-1,2 one-3 de formule:

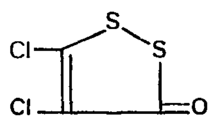

on isole le produit obtenu et soit on le transforme, le cas échéant, en un sel d'addition avec un acide, un sel métallique ou un sel d'addition avec une base azotée, soit, si on le désire, on déchlore le produit obtenu afin d'obtenir un produit de formule générale (I) dans laquelle R représente un atome d'hydrogène, puis isole le produit obtenu et le transforme, le cas échéant, en un sel d'addition avec un acide, un sel métallique ou un sel d'addition avec une base azotée.

2. Procédé selon la revendication 1, caractérisé en ce que la déchloration éventuelle est effectuée au moyen d'un hydrure de trialcoylétain.

3. Procédé selon l'une des revendications 1 ou 2, caractérisé en ce que la déchloration éventuelle est effectuée au moyen de l'hydrure de tributylétain.

**Patentansprüche für die Vertragsstaaten:**
BE, CH, DE, FR, GB, IT, LI, LU, NL, SE

1. Derivat des 5-Amino-1,2-dithiol-3-ons, dadurch gekennzeichnet, dass es der allgemeinen Formel

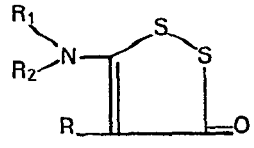

entspricht, worin R ein Wasserstoff- oder Chloratom bedeutet und
— entweder $R_1$ und $R_2$ zusammen mit dem Stickstoffatom, an das sie gebunden sind, einen 1,2,3,4-Tetrahydrochinoxalin-, 1,2,3,4-Tetrahydrochinolin-, 1,2,3,4-Tetrahydroisochinolin-, 3,4-Dihydro-2H-benzoxazin-1,4-, 2,3,4,5-Tetrahydro-1H-benzazepin- oder Isoindolin-Ring bilden, wobei alle diese Ringe gegebenenfalls einen oder mehrere Substituenten in irgendeiner Stellung tragen können, ausgewählt unter Halogenatomen und den Resten Carboxy, Alkyloxycarbonyl, Carbamoyl, Alkylcarbamoyl, Dialkylcarbamoyl, Trifluormethyl, Cyano, Nitro, Alkyloxy, Alkylthio oder Alkyl, gegebenenfalls substituiert durch einen Carboxy-, Alkyloxycarbonyl-, Carbamoyl-, Alkylcarbamoyl-, Dialkylcarbamoyl-, Hydroxy-, Amino-, Alkylamino- oder Dialkylamino-Rest,
— oder $R_1$ bedeutet einen Alkylrest und $R_2$ bedeutet einen Alkylrest, substituiert durch einen Phenylrest, der seinerseits nicht substituiert ist oder durch ein oder mehrere Atome oder Gruppen substituiert ist, ausgewählt unter den Halogenatomen und den Resten Alkyl, Alkoxy, Alkylthio, Hydroxy, Amino, Alkylamino, Dialkylamino, Carboxy, Alkyloxycarbonyl, Carbamoyl, Alkylcarbamoyl, Dialkylcarbamoyl, Trifluormethyl, Cyano, Nitro,
wobei alle vorstehend erwähnten Alkylreste und Alkylteile 1 bis 4 Kohlenstoffatome in gerader oder verzweigter Kette enthalten,
sowie seine Additionssalze mit Säuren, seine Metallsalze und seine Additionssalze mit Stickstoffbasen, wenn sie existieren.

2. Verfahren zur Herstellung eines Produkts gemäss Anspruch 1, in dessen Formel R ein Chloratom bedeutet und die übrigen Symbole wie in Anspruch 1 definiert sind, dadurch gekennzeichnet, dass man ein Amin der allgemeinen Formel

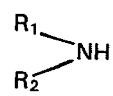

worin $R_1$ und $R_2$ wie in Anspruch 1 definiert sind, auf ein 4,5-Dichlor-1,2-thiol-3-on der Formel

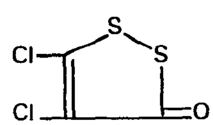

einwirken lässt, das erhaltene Produkt dann isoliert und es gegebenenfalls in ein Additionssalz mit einer Säure, ein Metallsalz oder ein Additionssalz mit einer Stickstoffbase überführt.

3. Verfahren zur Herstellung eines Produkts gemäss Anspruch 1, in dessen Formel R ein Wasserstoffatom bedeutet und die übrigen Symbole wie in Anspruch 1 definiert sind, dadurch gekennzeichnet, dass man ein Produkt der allgemeinen Formel (I), in dessen Formel R ein Chloratom darstellt und die übrigen Symbole wie in Anspruch 1 definiert sind, nämlich ein Produkt der allgemeinen Formel

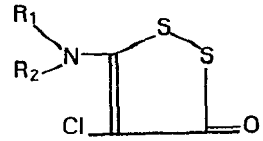

entchloriert, das erhaltene Produkt dann isoliert und es gegebenenfalls in ein Additionssalz mit einer Säure, ein Metallsalz oder ein Additionssalz mit einer Stickstoffbase überführt.

4. Verfahren gemäss Anspruch 3, dadurch gekennzeichnet, dass die Entchlorierung mittels eines Trialkylzinnhydrids bewirkt wird.

5. Verfahren gemäss einem der Ansprüche 3 oder 4, dadurch gekennzeichnet, dass die Entchlorierung mittels Tributylzinnhydrid bewirkt wird.

6. Pharmazeutische Zusammensetzung, dadurch gekennzeichnet, dass sie wenigstens ein Produkt gemäss Anspruch 1 enthält zusammen mit einem oder mehreren verträglichen und pharmazeutisch annehmbaren Verdünnungs- oder Hilfsmitteln.

**Patentansprüche für den Vertragsstaat:** AT

1. Verfahren zur Herstellung eines 5-Amino-1,2-dithiol-3-on-derivats der allgemeinen Formel

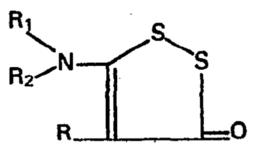

(I)

in welcher R ein Wasserstoff- oder Chloratom darstellt und

— $R_1$ und $R_2$ entweder zusammen mit dem Stickstoffatom, an das sie gebunden sind, einen 1,2,3,4-Tetrahydro-chinoxalin-, 1,2,3,4-Tetrahydro-chinolin-, 1,2,3,4-Tetrahydro-isochinolin-, 3,4-Dihydro-1,4-2H-benzoxazin-, 2,3,4,5-Tetrahydro-1H-Benzazepin- oder Isoindolinring bilden, wobei alle diese Ringe gegebenenfalls einen oder mehrere Substituenten, ausgewählt aus den Halogenatomen und den Carboxy-, Alkyloxycarbonyl-, Carbamoyl-, Alkylcarbamoyl-, Dialkylcarbamoyl-, Trifluormethyl-, Cyano-, Nitro-, Alkyloxy-, Alkylthioresten oder den gegebenenfalls durch einen Carboxy-, Alkyloxycarbonyl-, Carbamoyl-, Alkylcarbamoyl-, Dialkylcarbamoyl-, Hydroxy-, Amino-, Alkylamino- oder Dialkylaminorest, substituierten Alkylresten, in irgendeiner Position tragen können,

— oder $R_1$ stellt einen Alkylrest dar und $R_2$ stellt einen Alkylrest, substituiert durch einen selbst gegebenenfalls durch ein oder mehrere Atome oder Reste, ausgewählt aus den Halogenatomen und den Alkyl-, Alkyloxy-, Alkylthio-, Hydroxy-, Amino-, Alkylamino-, Dialkylamino-, Carboxy-, Alkyloxycarbonyl-, Carbamoyl-, Alkylcarbamoyl-, Dialkylcarbamoyl-, Trifluormethyl-, Cyano- oder Nitroresten, substituierten Phenylrest, dar,

wobei selbstverständlich alle oben genannten oder im folgenden erwähnten Alkylreste und Alkylteile 1 bis 4 Kohlenstoffatome in gerader oder verzweigter Kette enthalten,

sowie — falls existent — von ihren Säureadditionssalzen, ihren Metallsalzen und ihren Additionssalzen mit Stickstoffbasen, dadurch gekennzeichnet, dass man

zur Herstellung einer Verbindung der allgemeinen Formel (I), worin R ein Chloratom darstellt und die übrigen Symbole die obige Bedeutung haben, ein Amin der allgemeinen Formel:

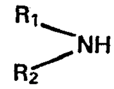

in welcher $R_1$ und $R_2$ die obige Bedeutung haben mit dem 4,5-Dichlor-1,2-dithiol-3-on der Formel

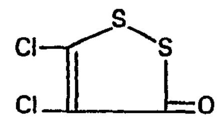

umsetzt, das erhaltene Produkt isoliert und es entweder zutreffendenfalls in ein Säureadditionssalz, ein Metallsalz oder ein Additionssalz mit einer Stickstoffbase umwandelt oder dass man gewünschtenfalls das erhaltene Produkt zur Herstellung einer Verbindung der allgemeinen Formel (I), worin R ein Wasserstoffatom darstellt, dechloriert, dann das erhaltene Produkt isoliert und es zutreffendenfalls in ein Säureadditionssalz, ein Metallsalz oder ein Additionssalz mit einer Stickstoffbase umwandelt.

2. Verfahren nach Anspruch 1, dadurch gekennzeichnet, dass die mögliche Dechlorierung mittels eines Trialkyletainhydrids ausgeführt wird.

3. Verfahren nach einem der Ansprüche 1 oder 2, dadurch gekennzeichnet, dass die mögliche Dechlorierung mittels Tributyletain ausgeführt wird.

**Claims for the contracting States:**
BE, CH, DE, FR, GB, IT, LI, LU, NL, SE

1. Derivative of 5-amino-1,2-dithiol-4-one, characterized in that it corresponds to the general formula:

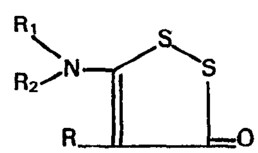

in which R represents a hydrogen or chlorine atom and

— either $R_1$ and $R_2$ together with the nitrogen atom to which they are bonded form a 1,2,3,4-tetrahydroquinoxaline, 1,2,3,4-tetrahydroquinoline, 1,2,3,4-tetrahydroisoquinoline, 3,4-dihydro-2H-benz-1,4-oxazine, 2,3,4,5-tetrahydro-1H-benzazepine or isoindoline ring, all of which rings can optionally carry, in any position, one or more substituents chosen from among halogen atoms and carboxyl, alkoxycarbonyl, carbamyl, alkylcarbamyl, dialkylcarbamyl, trifluoromethyl, cyano, nitro, alkoxy or alkylthio radicals or alkyl radicals optionally substituted by a carboxyl, alkoxycarbonyl, carbamyl, alkylcarbamyl, dialkylcarbamyl, hydroxyl, amino, alkylamino or dialkylamino radical,

— or $R_1$ represents an alkyl radical and $R_2$ represents an alkyl radical which is substituted by a phenyl radical which is itself unsubstituted or substituted by one or more atoms or radicals chosen from among halogen atoms and alkyl, alkoxy, alkylthio, hydroxyl, amino, alkylamino, dialkylamino, carboxyl, alkoxycarbonyl, carbamyl, alkylcarbamyl,

dialkylcarbamyl, trifluoromethyl, cyano or nitro radicals,
it being understood that all the alkyl radicals and alkyl portions referred to above contain 1 to 4 carbon atoms in a straight or branched chain,
as well as — where they exist — its addition salts with acids, its metal salts and its addition salts with nitrogen-containing bases.

2. Process for the preparation of a product according to Claim 1, in the formula of which R represents a chlorine atom and the other symbols are defined as in Claim 1, characterized in that an amine of the general formula:

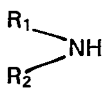

in which $R_1$ and $R_2$ are defined as in Claim 1, is reacted with 4,5-dichloro-1,2-dithiol-3-one of the formula:

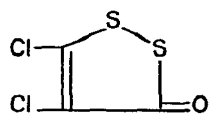

and that afterwards, the product obtained is isolated and converted, where appropriate, to an addition salt with an acid, metal salt or an addition salt with a nitrogen-containing base.

3. Process for the preparation of a product according to Claim 1, in the formula of which R represents a hydrogen atom and the other symbols are defined as in Claim 1, characterized in that a product of the general formula (I) in the formula of which R represents a chlorine atom and the other symbols are defined as in Claim 1, that is to say a product of the general formula:

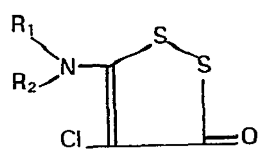

is dechlorinated and that afterwards, the product obtained is isolated and converted, where appropriate, to an addition salt with an acid, a metal salt or an addition salt with a nitrogen-containing base.

4. Process according to Claim 3, characterized in that the dechlorination is carried out by means of a trialkyl-tin hydride.

5. Process according to one of Claims 3 or 4, characterized in that the dechlorination is carried out by means of tributyl-tin hydride.

6. Pharmaceutical composition, characterized in that it contains at least one product according to Claim 1 together with one or more compatible and pharmaceutically acceptable diluents or adjuvants.

**Claims for the contracting State:** AT

1. Process for the preparation of a 5-amino-1,2-dithiol-3-one derivative of the general formula:

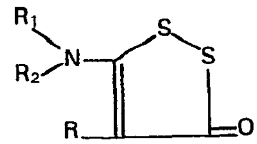

(I)

in which R represents a hydrogen or chlorine atom and

— either $R_1$ and $R_2$ together with the nitrogen atom to which they are bonded form a 1,2,3,4-tetrahydroquinoxaline, 1,2,3,4-tetrahydroquinoline, 1,2,3,4-tetrahydroisoquinoline, 3,4-dihydro-2H-benz-1,4-oxazine, 2,3,4,5-tetrahydro-1H-benzazepine or isoindoline ring, all of which rings can optionally carry, in any position, one or more substituents chosen from among halogen atoms and carboxyl, alkoxycarbonyl, carbamyl, alkylcarbamyl, dialkylcarbamyl, trifluoromethyl, cyano, nitro, alkoxy or alkylthio radicals or alkyl radicals optionally substituted by a carboxyl, alkoxycarbonyl, carbamyl, alkylcarbamyl, dialkylcarbamyl, hydroxyl, amino, alkylamino or dialkylamino radical,

— or $R_1$ represents an alkyl radical and $R_2$ represents an alkyl radical which is substituted by a phenyl radical which is itself unsubstituted or substituted by one or more atoms or radicals chosen from among halogen atoms and alkyl, alkoxy, alkylthio, hydroxyl, amino, alkylamino, dialkylamino, carboxyl, alkoxycarbonyl, carbamyl, alkylcarbamyl, dialkylcarbamyl, trifluoromethyl, cyano or nitro radicals,
it being understood that all the alkyl radicals and alkyl portions referred to above contain 1 to 4 carbon atoms in a straight or branched chain,
as well as — where they exist — its addition salts with acids, its metal salts and its addition salts with nitrogen-containing bases,
characterized in that, for the preparation of a product of the general formula (I) in which R represents a chlorine atom and the other symbols are defined as above, an amine of the general formula:

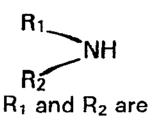

in which $R_1$ and $R_2$ are defined as above, is reacted with 4,5-dichloro-1,2-dithiol-3-one of the formula:

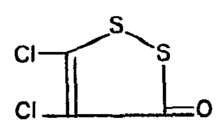

the product obtained is isolated and either converted, where appropriate to an addition salt with an acid, a metal salt or an addition salt with a nitrogen-containing base, or, where desired, dechlorinated so as to give a product of the general formula (I) in which R represents a hydrogen atom, after which this product is isolated and converted, where appropriate, to an addition salt with an acid, a metal salt or an addition salt with a nitrogen-containing base.

2. Process according to Claim 1, characterized in that the optional dechlorination is carried out by means of a trialkyl-tin hydride.

3. Process according to one of Claims 1 or 2, characterized in that the optional dechlorination is carried out by means of tributyl-tin hydride.